(19) Europäisches Patentamt
European Patent Office
Office européen des brevets

(11) Numéro de publication: **0 477 061 B1**

(12) **FASCICULE DE BREVET EUROPEEN**

(45) Date de publication de fascicule du brevet: **03.05.95**

(51) Int. Cl.⁶: **A61K 31/21**, A61K 9/20

(21) Numéro de dépôt: **91402377.5**

(22) Date de dépôt: **05.09.91**

(54) **Comprimé à libération prolongée à base de 5-mononitrate d'isosorbide et son procédé de préparation.**

(30) Priorité: **07.09.90 FR 9011133**

(43) Date de publication de la demande:
**25.03.92 Bulletin 92/13**

(45) Mention de la délivrance du brevet:
**03.05.95 Bulletin 95/18**

(84) Etats contractants désignés:
**AT BE CH DE DK ES FR GB GR IT LI LU NL SE**

(56) Documents cités:
**EP-A- 0 219 161**
**EP-A- 0 219 161**
**EP-A- 0 299 877**
**DE-A- 3 328 094**
**GB-A- 2 181 052**

(73) Titulaire: **PIERRE FABRE MEDICAMENT**
**45, Place Abel Gance**
**F-92100 Boulogne (FR)**

(72) Inventeur: **Bougaret, Joel**
**Plombières**
**F-81100 Castres (FR)**
Inventeur: **Sournac, Michel**
**15 rue Octave Terillon**
**F-21000 Dijon (FR)**

(74) Mandataire: **Ahner, Francis et al**
**CABINET REGIMBEAU**
**26, avenue Kléber**
**F-75116 Paris (FR)**

EP 0 477 061 B1

Rank Xerox (UK) Business Services
(3.10/3.09/3.3.3)

EP 0 477 061 B1

**Description**

La présente invention se rapporte à une composition pharmaceutique à base de 5-mononitrate d'isosorbide et ayant une forme galénique appropriée pour une libération prolongée dans le temps de ce principe actif.

Le 5-mononitrate d'isosorbide, (5-MNIS) métabolite actif de l'ISDN est le premier représentant d'une nouvelle génération de vasodilatateurs nitrés. Il permet un traitement préventif efficace de la maladie angineuse, et l'absence d'effet de premier passage hépatique lui confère des propriétés intéressantes dans l'insuffisance cardiaque.

Cependant le 5-MNIS présente des inconvénients qui rendent difficile sa mise sous forme galénique à libération prolongée c'est-à-dire :
- un phénomène d'électricité statique qui rend tout mélange à sec pratiquement impossible par suite d'une coulabilité quasi nulle,
- un phénomène de sublimation par exposition du 5 MNIS à des conditions particulières de stress (60°C - 75 % d'humidité relative) et,
- une très forte solubilité du 5-MNIS dans l'eau et les solutions tampons, qui rend difficile le contrôle de la libération du principe actif à partir de la forme galénique.

Le but de la présente invention est de mettre au point une forme galénique appropriée du 5-MNIS permettant de supprimer les inconvénients pré-cités.

Il a ainsi été mis en évidence qu'une fraction granulométrique bien déterminée du 5-MNIS sous forme d'une poudre présentait les qualités appropriées pour une telle formulation, c'est-à-dire peu de phénomènes électrostatiques, une tendance beaucoup moins nette à la sublimation et des qualités de coulabilité et compressibilité.

Plus précisément, la présente invention se rapporte à un comprimé caractérisé en ce qu'il comprend à titre de principe actif le 5-mononitrate d'isosorbide (5-MNIS) sous forme d'une poudre, de dimension granulométrique de 80 $\mu$m à 500 $\mu$m, déterminée par la méthode au tamis à jet d'air, en dispersion homogène dans une matrice hydrophile à base d'au moins un composant gonflant et d'au moins un composant diluant.

L'utilisation d'une poudre de telle granulométrie c'est-à-dire ayant des grains de dimension comprise entre 80 $\mu$m et 500 $\mu$m et de préférence ayant 70 % de ces grains de dimension comprise entre 80 $\mu$m et environ 250 $\mu$m présente, par ailleurs, un avantage supplémentaire. Elle permet de s'affranchir, lors de la fabrication de ladite composition, de la technique de granulation humide qui est une méthode courante mais présentant des inconvénients.

La méthode au tamis à jet d'air utilisée selon l'invention pour définir la granulométrie du 5-MNIS est une technique très utilisée par l'homme du métier en raison de sa grande sensibilité et fiabilité et lui est par conséquent très familière. Les conditions opératoires mises en oeuvre dans le cadre de l'invention seront explicitées plus en détail ci-après.

Le profil de granulométrie préféré pour le 5-MNIS selon l'invention et déterminé selon cette technique correspond à environ :
- 40 à 60 % de 5-MNIS de granulométrie supérieure à 80 $\mu$m et inférieure à 100 $\mu$m,
- 20 à 50 % de 5-MNIS supérieure à 100 $\mu$m et inférieure à 250 $\mu$m,
- environ 5 % de 5-MNIS supérieure à 250 $\mu$m et inférieure à 500 $\mu$m, et
- environ 0,5 % de granulométrie supérieure à 500 $\mu$m,
le résidu étant constitué de 5-MNIS présentant une granulométrie inférieure à 80 $\mu$m.

En ce qui concerne la matrice hydrophile, elle comprend au moins un composant gonflant et un composant diluant dans un rapport pondéral gonflant/diluant compris entre 0,2 et 0,7, de préférence égal à 0,5.

Le composant diluant comporte au moins un diluant intrinsèque et un diluant épaississant dans un rapport diluant épaississant/diluants compris entre 0,1 et 0,6, de préférence égal à 0,3.

Cette association permet de stabiliser la libération in vitro du 5-MNIS essentiellement durant l'étape terminale de la libération biphasique.

Selon la présente invention, le diluant intrinsèque est de préférence choisi parmi une ou plusieurs substances comprenant le lactose, le sorbitol, le mannitol, les phosphate ou sulfate de calcium, la silice colloïdale et/ou la cellulose microcristalline. Par contre ce diluant intrinsèque ne pourra être choisi parmi le polyvinylpyrrolidone et ses proches dérivés.

Le diluant épaississant est de préférence choisi parmi une ou plusieurs substances comprenant les amidons, les dérivés des amidons, la cellulose microfine, des gommes xanthanes naturelles ou hémisynthétiques et/ou les dextrines.

2

Quant au composant gonflant, il est de préférence choisi parmi une ou plusieurs substances polymères hydrophiles de viscosité apparente, à 2 % en poids par rapport au poids à 20°C, compris entre 0,1 Pa.s et 100 Pa.s. Ces substances de polymère hydrophile sont de préférence choisies dans la famille des hydrocolloïdes protéiques ou cellulosiques et, notamment parmi les dérivés alginiques, la méthylcellulose, la carboxyméthylcellulose, l'hydroxypropylcellulose et/ou la méthylhydroxypropylcellulose. Préférentiellement, la méthylhydroxypropylcellulose de viscosité voisine de 15 Pa.s sera utilisée.

Ces polymères pourront être utilisés seuls ou en association entre eux.

Grâce aux qualités intrinsèques du 5-MNIS ainsi sélectionné et aux caractéristiques de la forme matricielle hydrophile utilisée, il est ainsi obtenu une forme galénique présentant une excellente cohésion et caractérisée par une cinétique de libération in vitro, bi-phasique et indépendante du pH.

Les comprimés ainsi obtenus comportent de 5 % à 20 % en poids de principe actif par rapport au poids total du comprimé. Cette teneur sera de préférence égale à environ 10 % en poids du principe actif par rapport au poids total du comprimé.

Enfin selon un mode de réalisation préféré, on applique, sur la base matricielle précédemment évoquée, un pelliculage aqueux de préférence à base de dérivés cellulosiques compatibles, de type hydroxypropylcellulose ou méthylhydroxypropylcellulose, additionnés le cas échéant d'un agent plastifiant de type glycérine et/ou polyéthylèneglycols.

Ce pelliculage de matrices hydrophiles à l'aide de solvant aqueux s'avère répondre aux exigences du maintien de la résistance à l'écrasement, sans modification significative du profil de libération.

Ce même pelliculage peut bien entendu être coloré par l'adjonction de suspensions pigmentaires à préparation extemporanée.

Les comprimés selon l'invention pourront également contenir un agent lubrifiant tel l'acide stéarique ou dérivés et/ou un agent colorant de préférence de teinte rose sous la forme d'une laque organique de synthèse par exemple la laque érythrosine.

La présente invention se rapporte également à un procédé de fabrication desdits comprimés caractérisé en ce que :
- les différents composants gonflants et diluants et le principe actif sont tamisés puis mélangés ;
- on ajoute au mélange ainsi obtenu un lubrifiant et éventuellement d'autres additifs ;
- puis ce mélange final est comprimé et éventuellement,
- on effectue le pelliculage dudit comprimé ainsi obtenu.

Les comprimés ainsi obtenus pourront présenter une forme ovalaire éventuellement sécable.

Une telle formulation galénique du 5 mononitrate d'isosorbide présente donc les avantages de réduire le nombre de prises journalières, de diminuer les effets secondaires propres aux dérivés nitrés et liés à de trop fortes concentrations plasmatiques et par conséquent d'obtenir un taux plasmatique moyen stable sur plusieurs heures après administration. Un comprimé à base de 5-mononitrate d'isosorbide selon l'invention permet donc d'assurer une libération prolongée et régulière de ce principe actif pendant un intervalle de temps d'environ 6 à 18 heures.

Les comprimés obtenus selon l'invention sont particulièrement utiles pour le traitement de la maladie angineuse et des insuffisances cardiaques.

Les exemples et figures donnés ci-dessous à titre non limitatif permettront de mettre en évidence d'autres avantages et caractéristiques de la présente invention.

La figure 1 représente la cinétique de libération de 5-MNIS contenu dans une composition selon l'invention in vitro en fonction du temps.

La figure 2 représente la cinétique de libération du 5-MNIS contenu dans un comprimé selon l'invention in vitro en fonction de différents pH.

La figure 3 représente la courbe moyenne des taux plasmatiques de 5-MNIS obtenus après son administration sous forme de comprimés selon l'invention.

La figure 4 représente la courbe moyenne des pourcentages de 5-MNIS absorbés.

**EXEMPLE 1 :**

**Formulation de comprimés selon l'invention**

| comprimé n° | 1 | 2 | 3 | 4 | 5 |
|---|---|---|---|---|---|
| 5 mononitrate d'isosorbide | 50,0 | 40,0 | 20,0 | 80,0 | 60,0 |
| lactose | 140,0 | 170,0 | 60,0 | 200,0 | 200,0 |
| mannitol | 50,0 | | | | |
| amidon de maïs prégélatinisé | | 69,0 | 50,0 | | 75,0 |
| gomme xanthane | 60,0 | | | 100,0 | |
| MHPC 4 Pa.s | | | 20,0 | | |
| MHPC 15 Pa.s | 107,0 | 115,0 | 40,0 | 150,0 | 100,0 |
| Stéarate de Magnésium | 4,35 | 3,88 | 2,6 | 4,45 | 4 |
| Silice colloïdale anhydre | 2,5 | 2,0 | 1,34 | 2,35 | 2,5 |
| Laque érythrosine E 127 | 0,15 | 0,12 | 0,06 | 0,2 | |
| Masse Unitaire intermédiaire | 414,0 | 400,0 | 194,0 | 537,0 | 441,5 |
| MHPC partiellement substituée Polyoxyl 8 stéarate | 13,95 | 13,955 | 5,975 | 11,65 | |
| Laurylsulfate de sodium | 0,05 | 0,045 | 0,025 | 0,05 | |
| Glycérine Codex | 2,0 | 2,0 | 1,0 | 2,3 | |
| Masse Unitaire finale | 430,0 | 416,0 | 201,0 | 551,0 | 441,5 |

**EXEMPLE 2 :**

**Caractérisation de la granulométrie du 5-MNIS utilisé selon l'invention par la méthode au tamis à jet d'air de type ALPIN JET SIEVE**

conditions opératoires
    . échantillon de 20 g,
    . 350 g dépression d'air,
    . durée 4 mn de 80 à 100 $\mu$m puis 3 mm de 250 à 500 $\mu$m.
    Dans ces conditions, il a été testé trois lots de 5-MNIS, A, B, et C. Les résultats sont présentés dans le tableau suivant :

| Profil recherché | | A | B | C |
|---|---|---|---|---|
| | | % | % | % |
| 80 µm | 40 ⟨ ⟨ 60 | 47,5 | 48,2 | 49,2 |
| 100 µm | 20 ⟨ ⟨ 50 | 26 | 34,4 | 34,85 |
| 250 µm | ⟨ 5 | 0,0 | 0,9 | 0,0 |
| 500 µm | ⟨ 0,5 | 0,0 | 0,0 | 0,0 |

Ces trois lots présentent bien le profil granulométrique recherché.

**EXEMPLE 3 :**

**Cinétiques de libération in vitro**

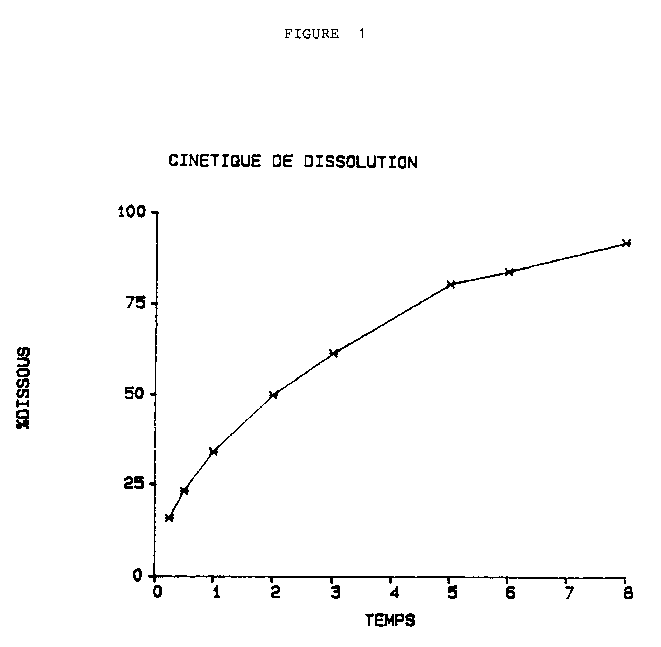

L'étude de l'évolution du pourcentage de 5-MNIS dissous a été effectuée en fonction du temps. Cet essai a été réalisé à l'aide de l'appareil à palettes tournantes (100 rpm/900 ml d'eau). Les résultats obtenus sont les suivants et sont représentés en figure 1 :

| - % dissous à 0,25 heure | 5 % à 20 %, |
|---|---|
| - % dissous à 1 heure | 20 % à 40 % |
| - % dissous à 4 heures | 60% à 80 % |
| - % dissous à 8 heures | $\geq$ 80 % |

**EXEMPLE 4 :**

**Cinétiques de libération in vitro du principe actif en fonction du pH**

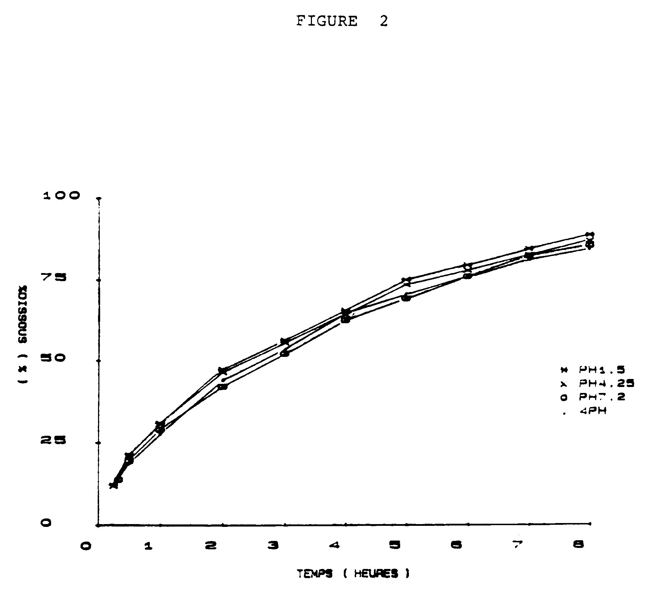

Cette étude a été réalisée à des valeurs de pH de 1,5, 4,25, 7,2 et variables. Les résultats obtenus sont représentés en figure 2.

Les trois cinétiques de libération à pH constant, ne diffèrent pas significativement entre elles. Quant au profil de dissolution obtenu à pH variable, en se superposant aux profils précédents, il atteste d'un mode de libération indépendant du pH.

**EXEMPLE 5 :**

**Caractéristiques pharmacocinétiques**

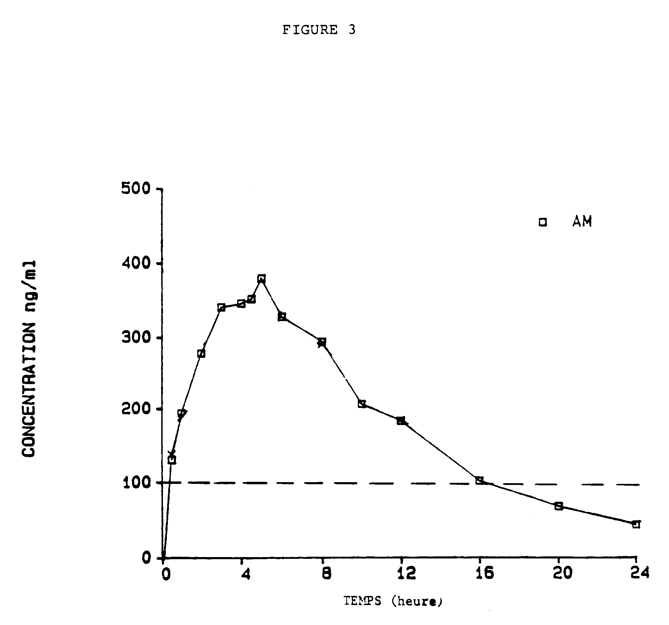

La figure 3 représente la courbe moyenne des taux plasmatiques de 5-MNIS observés après administration chez 3 sujets, à dose unique d'un comprimé n° 2 ; les paramètres pharmacocinétiques calculés lors de cette étude pilote de screening sont les suivants :

|  | Tmax | Cmax | T 1/2 | MRT | $AUC_{0-\infty}$ |
|---|---|---|---|---|---|
|  | h | $ng.ml^{-1}$ | h | h | $ng.ml^{-1}.h$ |
| MOY. | 4,67 | 386,27 | 4,72 | 10,02 | 4670,33 |
| S.D | 1,53 | 35,44 | 2,06 | 1,46 | 470,08 |

Sur cette courbe plasmatique moyenne, compte tenu de l'existence d'une valeur de concentration minimale relatée efficace de 100 ng. $ml^{-1}$ (= $C_{eff}$), on note le maintien des taux plasmatiques au delà de cette valeur, sans temps de latence significatif, pendant un intervalle de temps t C $C_{eff}$ voisin de 16 heures.

**EXEMPLE 6 :**

**Profil d'absorption in vivo**

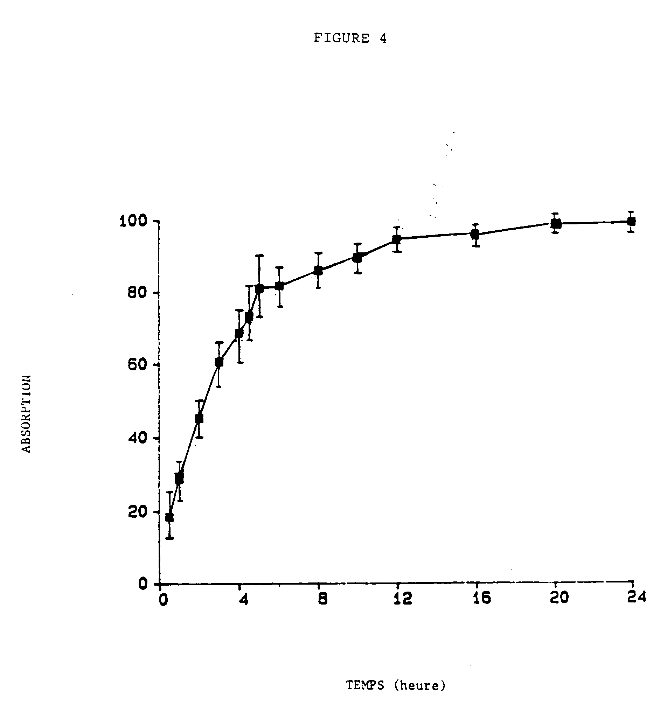

La figure 4 représente la courbe moyenne des % de 5-MNIS absorbés. Le profil d'absorption obtenu est caractérisé par une entrée d'ordre zéro jusqu'à environ la cinquième heure suivant la prise ; à ce titre le flux moyen constant d'absorption in vivo, calculé à partir de cette courbe est de l'ordre de 13,37 % $h^{-1}$.

**Revendications**
**Revendications pour les Etats contractants suivants : AT, BE, CH, DE, DK, FR, GB, IT, LI, LU, NL, SE**

1. Comprimé à libération prolongée, caractérisé en ce qu'il comprend à titre de principe actif le 5-mononitrate d'isosorbide (5-MNIS), sous forme d'une poudre présentant une granulométrie de 80 $\mu$m à 500 $\mu$m, déterminée selon la méthode du tamis a jet d'air, et dont au moins 70 % des grains ont une dimension comprise entre 80 $\mu$m et environ 250 $\mu$m, en dispersion homogène dans une matrice hydrophile à base d'au moins un composant gonflant et d'au moins un composant diluant, la polyvinylpyrrolidone étant exclue dudit comprimé.

2. Comprimé à libération prolongée selon la revendication 1, caractérisé en ce que la poudre de 5-mononitrate présente de préférence le profil granulométrique suivant :
   - 40 à 60 % de 5-MNIS de granulométrie supérieure à 80 $\mu$m et inférieure à 100 $\mu$m,
   - 20 à 50 % de 5-MNIS supérieure à 100 $\mu$m et inférieure a 250 $\mu$m,
   - environ 5 % de 5-MNIS supérieure a 250 $\mu$m et inférieure à 500 $\mu$m, et
   - environ 0,5 % de granulométrie supérieure à 500 $\mu$m,
   le résidu étant constitué de 5-MNIS présentant une granulométrie inférieure à 80 $\mu$m.

3. Comprimé à libération prolongée selon l'une des revendications 1 ou 2, caractérisé en ce que le principe actif est présent à raison de 5 à 20 % en poids et de préférence a raison d'environ 10 % en poids par rapport au poids total dudit comprimé.

4. Comprimé à libération prolongée selon l'une des revendications 1 à 3, caractérisé en ce que le composant gonflant et le composant diluant sont présents dans ledit comprimé selon un rapport pondéral composant gonflant/composant diluant compris entre 0,2 et 0,7 et de préférence égal à environ 0,5.

5. Comprimé à libération prolongée selon la revendication 4, caractérisé en ce que le composant gonflant comporte une ou plusieurs substances polymères hydrophiles de viscosité apparente comprise entre 0,1 et 100 Pa.s.

**6.** Comprimé selon la revendication 5, caractérisé en ce que les substances polymères hydrophiles sont choisies dans la famille des hydrocolloïdes protéiques ou cellulosiques et de préférence parmi les dérivés alginiques, les gommes naturelles, la méthylcellulose, la carboxyméthylcellulose, l'hydroxypropylcellulose et/ou l'éthylhydroxypropylcellulose.

**7.** Comprimé à libération prolongée selon la revendication 6, caractérisé en ce que la substance polymère est de préférence la méthylhydroxypropylcellulose de viscosité voisine de 15 Pa.s.

**8.** Comprimé à libération prolongée selon l'une des revendications 1 à 7, caractérisé en ce que le composant diluant comporte au moins un diluant intrinsèque et un diluant épaississant.

**9.** Comprimé à libération prolongée selon la revendication 8, caractérisé en ce que le rapport composant diluant épaississant/composants diluants est compris entre 0,1 et 0,6 et de préférence égal à environ 0,3.

**10.** Comprimé à libération prolongée selon la revendication 8 ou 9, caractérisé en ce que le diluant intrinsèque est de préférence choisi parmi le lactose, le sorbitol, le mannitol, les phosphate ou sulfate de calcium, la silice colloïdale et/ou la cellulose microcristalline.

**11.** Comprimé à libération prolongée selon la revendication 8 ou 9, caractérisé en ce que le diluant épaississant est de préférence choisi parmi des amidons, des dérivés d'amidons, des gommes xanthane, de la cellulose microfine et/ou des dextrines.

**12.** Comprimé à libération prolongée selon l'une des revendications 1 à 11, caractérisé en ce qu'il comprend en outre un pelliculage aqueux à base de dérivés cellulosiques.

**13.** Comprimé à libération prolongée selon l'une des revendications 1 à 12, caractérisé en ce que le comprimé comprend également d'autres additifs choisis parmi les lubrifiants, ou substances colorantes.

**14.** Procédé de fabrication d'un comprimé selon l'une des revendications 1 à 13, caractérisé en ce qu'on réalise :
- le tamisage des différents composants gonflants et diluants et du principe actif,
- le mélange de ces composants et du principe actif,
- l'ajout d'un lubrifiant et éventuellement d'autres additifs à ce mélange,
- la compression du mélange ainsi obtenu et éventuellement,
- le pelliculage dudit comprimé.

**Revendications pour les Etats contractants suivants : ES, GR**

**1.** Procédé de préparation d'un comprimé à libération prolongée, caractérisé en ce qu'on réalise :
- le tamisage des différents composants gonflants et diluants et du principe actif,
- le mélange de ces composants et du principe actif,
- l'ajout d'un lubrifiant et éventuellement d'autres additifs à ce mélange,
- la compression du mélange ainsi obtenu et éventuellement,
- le pelliculage dudit comprimé,

et en ce que le comprimé comprend à titre de principe actif le 5-mononitrate d'isosorbide (5-MNIS), sous forme d'une poudre présentant une granulométrie de 80 $\mu$m à 500 $\mu$m, déterminée selon la méthode du tamis à jet d'air, et dont au moins 70 % des grains ont une dimension comprise entre 80 $\mu$m et environ 250 $\mu$m, en dispersion homogène dans une matrice hydrophile à base d'au moins un composant gonflant et d'au moins un composant diluant, la polyvinylpyrrolidone étant exclue dudit comprimé.

**2.** Procédé de préparation d'un comprimé à libération prolongée selon la revendication 1, caractérisé en ce que la poudre de 5-mononitrate présente de préférence le profil granulométrique suivant :
- 40 à 60 % de 5-MNIS de granulométrie supérieure à 80 $\mu$m et inférieure à 100 $\mu$m,
- 20 à 50 % de 5-MNIS supérieure à 100 $\mu$m et inférieure a 250 $\mu$m,
- environ 5 % de 5-MNIS supérieure à 250 $\mu$m et inférieure à 500 $\mu$m, et

- environ 0,5 % de granulométrie supérieure à 500 $\mu$m,

le résidu étant constitué de 5-MNIS présentant une granulométrie inférieure à 80 $\mu$m.

3. Procédé de préparation d'un comprimé à libération prolongée selon l'une des revendications 1 ou 2, caractérisé en ce que le principe actif est présent à raison de 5 à 20 % en poids et de préférence à raison d'environ 10 % en poids par rapport au poids total dudit comprimé.

4. Procédé de préparation d'un comprimé à libération prolongée selon l'une des revendications 1 à 3, caractérisé en ce que le composant gonflant et le composant diluant sont présents dans ledit comprimé selon un rapport pondéral composant gonflant/composant diluant compris entre 0,2 et 0,7 et de préférence égal a environ 0,5.

5. Procédé de préparation d'un comprimé à libération prolongée selon la revendication 4, caractérisé en ce que le composant gonflant comporte une ou plusieurs substances polymères hydrophiles de viscosité apparente comprise entre 0,1 et 100 Pa.s.

6. Procédé de préparation d'un comprimé à libération prolongée selon la revendication 5, caractérisé en ce que les substances polymères hydrophiles sont choisies dans la famille des hydrocolloïdes protéiques ou cellulosiques et de préférence parmi les dérivés alginiques, les gommes naturelles, la méthylcellulose, la carboxyméthylcellulose, l'hydroxypropylcellulose et/ou l'éthylhydroxypropylcellulose.

7. Procédé de préparation d'un comprimé à libération prolongée selon la revendication 6, caractérisé en ce que la substance polymère est de préférence la méthylhydroxypropylcellulose de viscosité voisine de 15 Pa.s.

8. Procédé de préparation d'un comprimé à libération prolongée selon l'une des revendications 1 à 7, caractérisé en ce que le composant diluant comporte au moins un diluant intrinsèque et un diluant épaississant.

9. Procédé de préparation d'un comprimé à libération prolongée selon la revendication 8, caractérisé en ce que le rapport composant diluant épaississant/composants diluants est compris entre 0,1 et 0,6 et de préférence égal à environ 0,3.

10. Procédé de préparation d'un comprimé à libération prolongée selon la revendication 8 ou 9, caractérise en ce que le diluant intrinsèque est de préférence choisi parmi le lactose, le sorbitol, le mannitol, les phosphate ou sulfate de calcium, la silice colloïdale et/ou la cellulose microcristalline.

11. Procédé de préparation d'un comprimé à libération prolongée selon la revendication 8 ou 9, caractérisé en ce que le diluant épaississant est de préférence choisi parmi des amidons, des dérivés d'amidons, des gommes xanthane, de la cellulose microfine et/ou des dextrines.

12. Procédé de préparation d'un comprimé à libération prolongée selon l'une des revendications 1 à 11, caractérisé en ce qu'il comprend en outre un pelliculage aqueux à base de dérivés cellulosiques.

13. Procédé de préparation d'un comprimé à libération prolongée selon l'une des revendications 1 à 12, caractérisé en ce que le comprimé comprend également d'autres additifs choisis parmi les lubrifiants, ou substances colorantes.

**Claims**

**Claims for the following Contracting States : AT, BE, CH, DE, DK, FR, GB, IT, LI, LU, NL, SE**

1. Sustained-release tablet, characterized in that it comprises as active principle isosorbide 5-mononitrate (5-ISMN) in the form of a powder having a particle size of 80 $\mu$m to 500 $\mu$m, determined according to the air-jet sieve method, and in which at least 70% of the particles are between 80 $\mu$m and approximately 250 $\mu$m in size, in homogeneous dispersion in a hydrophilic matrix based on at least one swelling component and at least one diluent component, polyvinylpyrrolidone being excluded from the said tablet.

2. Sustained-release tablet according to Claim 1, characterized in that the 5-mononitrate powder preferably has the following particle size profile:
   - 40 to 60% of 5-ISMN of particle size larger than 80 $\mu$m and smaller than 100 $\mu$m,
   - 20 to 50% of 5-ISMN larger than 100 $\mu$m and smaller than 250 $\mu$m,
   - approximately 5% of 5-ISMN larger than 250 $\mu$m and smaller than 500 $\mu$m, and
   - approximately 0.5% of particle size larger than 500 $\mu$m,
   the residue consisting of 5-ISMN having a particle size smaller than 80 $\mu$m.

3. Sustained-release tablet according to either of Claims 1 and 2, characterized in that the active principle is present in the proportion of 5 to 20% by weight, and preferably in the proportion of approximately 10% by weight, relative to the total weight of the said tablet.

4. Sustained-release tablet according to one of Claims 1 to 3, characterized in that the swelling component and the diluent component are present in the said tablet according to a swelling component/diluent component weight ratio of between 0.2 and 0.7, and preferably equal to approximately 0.5.

5. Sustained-release tablet according to Claim 4, characterized in that the swelling component contains one or more hydrophilic polymeric substances of apparent viscosity between 0.1 and 100 Pa.s.

6. Tablet according to Claim 5, characterized in that the hydrophilic polymeric substances are chosen from the family of proteinaceous or cellulosic hydrocolloids, and preferably from alginic derivatives, natural gums, methylcellulose, carboxymethylcellulose, hydroxypropylcellulose and/or ethylhydroxypropylcellulose.

7. Sustained-release tablet according to Claim 6, characterized in that the polymeric substance is preferably methylhydroxypropylcellulose of viscosity in the region of 15 Pa.s.

8. Sustained-release tablet according to one of Claims 1 to 7, characterized in that the diluent component contains at least one intrinsic diluent and one thickening diluent.

9. Sustained-release tablet according to Claim 8, characterized in that the thickening diluent component/diluent components ratio is between 0.1 and 0.6, and preferably equal to approximately 0.3.

10. Sustained-release tablet according to Claim 8 or 9, characterized in that the intrinsic diluent is preferably chosen from lactose, sorbitol, mannitol, calcium phosphate or sulfate, colloidal silica and/or microcrystalline cellulose.

11. Sustained-release tablet according to Claim 8 or 9, characterized in that the thickening diluent is preferably chosen from starches, starch derivatives, xanthan gums, microfine cellulose and/or dextrins.

12. Sustained-release tablet according to one of Claims 1 to 11, characterized in that it comprises, in addition, an aqueous film-coating based on cellulose derivatives.

13. Sustained-release tablet according to one of Claims 1 to 12, characterized in that the tablet also comprises other additives chosen from lubricants or colouring substances.

14. Process for manufacturing a tablet according to one of Claims 1 to 13, characterized in that the following are carried out:
   - sieving of the various swelling and diluent components and of the active principle,
   - mixing of these components and the active principle,
   - addition of a lubricant and, where appropriate, other additives to this mixture,
   - tableting of the mixture thereby obtained and, where appropriate,
   - film-coating of the said tablet.

**Claims for the following Contracting States : ES, GR**

1. Process for preparing a sustained-release tablet, characterized in that the following are carried out:
   - sieving of the various swelling and diluent components and of the active principle,

- mixing of these components and the active principle,
- addition of a lubricant and, where appropriate, other additives to this mixture,
- tableting of the mixture thereby obtained and, where appropriate,
- film-coating of the said tablet,

and in that the tablet comprises as active principle isosorbide 5-mononitrate (5-ISMN) in the form of a powder having a particle size of 80 $\mu$m to 500 $\mu$m, determined according to the air-jet sieve method, and in which at least 70% of the particles are between 80 $\mu$m and approximately 250 $\mu$m in size, in homogeneous dispersion in a hydrophilic matrix based on at least one swelling component and at least one diluent component, polyvinylpyrrolidone being excluded from the said tablet.

2. Process for preparing a sustained-release tablet according to Claim 1, characterized in that the 5-mononitrate powder preferably has the following particle size profile:
   - 40 to 60% of 5-ISMN of particle size larger than 80 $\mu$m and smaller than 100 $\mu$m,
   - 20 to 50% of 5-ISMN larger than 100 $\mu$m and smaller than 250 $\mu$m,
   - approximately 5% of 5-ISMN larger than 250 $\mu$m and smaller than 500 $\mu$m, and
   - approximately 0.5% of particle size larger than 500 $\mu$m,
   the residue consisting of 5-ISMN having a particle size smaller than 80 $\mu$m.

3. Process for preparing a sustained-release tablet according to either of Claims 1 and 2, characterized in that the active principle is present in the proportion of 5 to 20% by weight, and preferably in the proportion of approximately 10% by weight, relative to the total weight of the said tablet.

4. Process for preparing a sustained-release tablet according to one of Claims 1 to 3, characterized in that the swelling component and the diluent component are present in the said tablet according to a swelling component/diluent component weight ratio of between 0.2 and 0.7, and preferably equal to approximately 0.5.

5. Process for preparing a sustained-release tablet according to Claim 4, characterized in that the swelling component contains one or more hydrophilic polymeric substances of apparent viscosity between 0.1 and 100 Pa.s.

6. Process for preparing a sustained-release tablet according to Claim 5, characterized in that the hydrophilic polymeric substances are chosen from the family of proteinaceous or cellulosic hydrocolloids, and preferably from alginic derivatives, natural gums, methylcellulose, carboxymethylcellulose, hydroxypropylcellulose and/or ethylhydroxypropylcellulose.

7. Process for preparing a sustained-release tablet according to Claim 6, characterized in that the polymeric substance is preferably methylhydroxypropylcellulose of viscosity in the region of 15 Pa.s.

8. Process for preparing a sustained-release tablet according to one of Claims 1 to 7, characterized in that the diluent component contains at least one intrinsic diluent and one thickening diluent.

9. Process for preparing a sustained-release tablet according to Claim 8, characterized in that the thickening diluent component/diluent components ratio is between 0.1 and 0.6, and preferably equal to approximately 0.3.

10. Process for preparing a sustained-release tablet according to Claim 8 or 9, characterized in that the intrinsic diluent is preferably chosen from lactose, sorbitol, mannitol, calcium phosphate or sulfate, colloidal silica and/or microcrystalline cellulose.

11. Process for preparing a sustained-release tablet according to Claim 8 or 9, characterized in that the thickening diluent is preferably chosen from starches, starch derivatives, xanthan gums, microfine cellulose and/or dextrins.

12. Process for preparing a sustained-release tablet according to one of Claims 1 to 11, characterized in that it comprises, in addition, an aqueous film-coating based on cellulose derivatives.

**13.** Process for preparing a sustained-release tablet according to one of Claims 1 to 12, characterized in that the tablet also comprises other additives chosen from lubricants or colouring substances.

**Patentansprüche**

**Patentansprüche für folgende Vertragsstaaten : AT, BE, CH, DE, DK, FR, GB, IT, LI, LU, NL, SE**

**1.** Tablette mit anhaltender Freisetzung (mit Depot-Wirkung) dadurch gekennzeichnet, daß sie als Wirkstoff das Isosorbid-5-mononitrat (5-MNIS) in Form eines Pulvers mit einer Korngröße von 80 bis 500 μm, bestimmt nach dem Luftstrahlsiebverfahren, in dem mindestens 70 % der Körnchen eine Dimension zwischen 80 μm und etwa 250 μm haben, in homogen dispergierter Form in einer hydrophilen Matrix auf Basis mindestens einer Quellmittel-Komponente und mindestens einer Verdünnungsmittel-Komponente enthält, und die kein Polyvinylpyrrolidon enthält.

**2.** Tablette mit anhaltender Freisetzung nach Anspruch 1, dadurch gekennzeichnet, daß das 5-Mononitrat-Pulver vorzugsweise die folgende Korngrößen-Verteilung aufweist:
   - 40 bis 60 % von 5-MNIS haben eine Korngröße von über 80 μm und unter 100 μm,
   - 20 bis 50 % von 5-MNIS haben eine Korngröße von über 100 μm und unter 250 μm,
   - etwa 5 % von 5-MNIS haben eine Korngröße von über 250 μm und unter 500 μm und
   - etwa 0,5 % von 5-MNIS haben eine Korngröße von über 500 μm,

   wobei der Rest aus 5-MNIS mit einer Korngröße von unter 80 μm besteht.

**3.** Tablette mit anhaltender Freisetzung nach einem der Ansprüche 1 oder 2, dadurch gekennzeichnet, daß der Wirkstoff in einer Menge von 5 bis 20 Gew.-%, vorzugsweise von etwa 10 Gew.-%, bezogen auf das Gesamtgewicht der Tablette, vorliegt.

**4.** Tablette mit anhaltender Freisetzung nach einem der Ansprüche 1 bis 3, dadurch gekennzeichnet, daß die Quellmittel-Komponente und die Verdünnungsmittel-Komponente in der genannten Tablette in einem Gewichtsverhältnis von Quellmittel-Komponente/Verdünnungsmittel-Komponente zwischen 0,2 und 0,7, vorzugsweise von etwa 0,5, vorliegen.

**5.** Tablette mit anhaltender Freisetzung nach Anspruch 4, dadurch gekennzeichnet, daß die Quellmittel-Komponente eine oder mehr hydrophile polymere Substanzen mit einer scheinbaren Viskosität zwischen 0,1 und 100 Pa.s umfaßt.

**6.** Tablette nach Anspruch 5, dadurch gekennzeichnet, daß die hydrophilen polymeren Substanzen ausgewählt werden aus der Familie der Protein- oder Cellulose-Hydrokolloide und vorzugsweise aus den Alginsäurederivaten, natürlichen Gummis (Harzen), Methylcellulose, Carboxymethylcellulose, Hydroxypropylcellulose und/oder Ethylhydroxypropylcellulose.

**7.** Tablette mit anhaltender Freisetzung nach Anspruch 6, dadurch gekennzeichnet, daß die polymere Substanz vorzugsweise Methylhydroxypropylcellulose mit einer Viskosität von etwa 15 Pa.s ist.

**8.** Tablette mit anhaltender Freisetzung nach einem der Ansprüche 1 bis 7, dadurch gekennzeichnet, daß die Verdünnungsmittel-Komponente mindestens ein echtes Verdünnungsmittel und ein eindickendes Verdünnungsmittel umfaßt.

**9.** Tablette mit anhaltender Freisetzung nach Anspruch 8, dadurch gekennzeichnet, daß das Verhältnis zwischen der eindickenden Verdünnungsmittel-Komponente und den Verdünnungsmittel-Komponentenzwischen 0,1 und 0,6, vorzugsweise bei etwa 0,3, liegt.

**10.** Tablette mit anhaltender Freisetzung nach Anspruch 8 oder 9, dadurch gekennzeichnet, daß das echte Verdünnungsmittel Vorzugsweise ausgewählt wird aus Lactose, Sorbit, Mannit, Calciumphosphat oder Calciumsulfat, kolloidalem Siliciumdioxid und/oder mikrokristalliner Cellulose.

**11.** Tablette mit anhaltender Freisetzung nach Anspruch 8 oder 9, dadurch gekennzeichnet, daß das eindickende Verdünnungsmittel vorzugsweise ausgewählt wird aus Stärken, Stärkederivaten, Xanthangummis, mikrofeiner Cellulose und/oder Dextrinen.

**12.** Tablette mit anhaltender Freisetzung nach einem der Ansprüche 1 bis 11, dadurch gekennzeichnet, daß sie außerdem einen wäßrigen Filmüberzug auf Basis von Cellulosederivaten enthält.

**13.** Tablette mit anhaltender Freisetzung nach einem der Ansprüche 1 bis 12, dadurch gekennzeichnet, daß die Tablette außerdem Zusätze enthält, die ausgewählt werden aus Gleitmitteln (Schmiermitteln) oder färbenden Substanzen.

**14.** Verfahren zur Herstellung einer Tablette nach einem der Ansprüche 1 bis 13, dadurch gekennzeichnet, daß man
- die verschiedenen Quellmittel- und Verdünnungsmittel-Komponenten und den Wirkstoff siebt,
- diese Komponenten und den Wirkstoff miteinander mischt,
- ein Gleitmittel (Schmiermittel) und gegebenenfalls weitere Additive dieser Mischung zusetzt,
- die so erhaltene Mischung komprimiert (preßt) und gegebenenfalls
- die Tablette mit einem Filmüberzug versieht.

**Patentansprüche für folgende Vertragsstaaten : ES, GR**

**1.** Verfahren zur Herstellung einer Tablette mit anhaltender Freisetzung (mit Depotwirkung), dadurch gekennzeichnet, daß man
- die verschiedenen Quellmittel- und Verdünnungsmittel-Komponenten und den Wirkstoff siebt,
- diese Komponenten und den Wirkstoff miteinander mischt,
- ein Gleitmittel (Schmiermittel) und gegebenenfalls weitere Additive dieser Mischung zusetzt,
- die so erhaltene Mischung komprimiert (preßt) und gegebenenfalls
- die Tablette mit einem Filmüberzug versieht, und

daß die Tablette als Wirkstoff das Isosorbid-5-mononitrat (5-MNIS) in Form eines Pulvers mit einer Korngröße von 80 bis 500 $\mu$m, bestimmt nach dem Luftstrahlsiebverfahren, in dem mindestens 70 % der Körnchen eine Dimension zwischen 80 $\mu$m und etwa 250 $\mu$m haben, in homogen dispergierter Form in einer hydrophilen Matrix auf Basis mindestens einer Quellmittel-Komponente und mindestens einer Verdünnungsmittel-Komponente enthält, und die kein Polyvinylpyrrolidon enthält.

**2.** Verfahren zur Herstellung einer Tablette mit anhaltender Freisetzung nach Anspruch 1, dadurch gekennzeichnet, daß das 5-Mononitrat-Pulver vorzugsweise die folgende Korngrößenverteilung aufweist:
- 40 bis 60 % von 5-MNIS haben eine Korngröße von über 80 $\mu$m und unter 100 $\mu$m,
- 20 bis 50 % von 5-MNIS haben eine Korngröße von über 100 $\mu$m und unter 250 $\mu$m,
- etwa 5 % von 5-MNIS haben eine Korngröße von über 250 $\mu$m und unter 500 $\mu$m und
- etwa 0,5 % von 5-MNIS haben eine Korngröße von über 500 $\mu$m,

wobei der Rest aus 5-MNIS mit einer Korngröße von unter 80 $\mu$m besteht.

**3.** Verfahren zur Herstellung einer Tablette mit anhaltender Freisetzung nach einem der Ansprüche 1 oder 2, dadurch gekennzeichnet, daß der Wirkstoff in einer Menge von 5 bis 20 Gew.-%, vorzugsweise von etwa 10 Gew.-%, bezogen auf das Gesamtgewicht der Tablette, vorliegt.

**4.** Verfahren zur Herstellung einer Tablette mit anhaltender Freisetzung nach einem der Ansprüche 1 bis 3, dadurch gekennzeichnet, daß die Quellmittel-Komponente und die Verdünnungsmittel-Komponente in der genannten Tablette in einem Gewichtsverhältnis von Quellmittel-Komponente/Verdünnungsmittel-Komponente zwischen 0,2 und 0,7, vorzugsweise von etwa 0,5, vorliegen.

**5.** Verfahren zur Herstellung einer Tablette mit anhaltender Freisetzung nach Anspruch 4, dadurch gekennzeichnet, daß die Quellmittel-Komponente eine oder mehr hydrophile polymere Substanzen mit einer scheinbaren Viskosität zwischen 0,1 und 100 Pa.s umfaßt.

**6.** Verfahren zur Herstellung einer Tablette mit anhaltender Freisetzung nach Anspruch 5, dadurch gekennzeichnet, daß die hydrophilen polymeren Substanzen ausgewählt werden aus der Familie der Protein- oder Cellulose-Hydrokolloide und vorzugsweise aus den Alginsäurederivaten, natürlichen Gummis (Harzen), Methylcellulose, Carboxymethylcellulose, Hydroxypropylcellulose und/oder Ethylhydroxypropylcellulose.

7. Verfahren zur Herstellung einer Tablette mit anhaltender Freisetzung nach Anspruch 6, dadurch gekennzeichnet, daß die polymere Substanz vorzugsweise Methylhydroxypropylcellulose mit einer Viskosität von etwa 15 Pa.s ist.

8. Verfahren zur Herstellung einer Tablette mit anhaltender Freisetzung nach einem der Ansprüche 1 bis 7, dadurch gekennzeichnet, daß die Verdünnungsmittel-Komponente mindestens ein echtes Verdünnungsmittel und ein eindickendes Verdünnungsmittel umfaßt.

9. Verfahren zur Herstellung einer Tablette mit anhaltender Freisetzung nach Anspruch 8, dadurch gekennzeichnet, daß das Verhältnis zwischen der eindickenden Verdünnungsmittel-Komponente und den Verdünnungsmittel-Komponenten zwischen 0,1 und 0,6, vorzugsweise bei etwa 0,3, liegt.

10. Verfahren zur Herstellung einer Tablette mit anhaltender Freisetzung nach Anspruch 8 oder 9, dadurch gekennzeichnet, daß das echte Verdünnungsmittel vorzugsweise ausgwählt wird aus Lactose, Sorbit, Mannit, Calciumphosphat oder Calciumsulfat, kolloidalem Siliciumdioxid und/oder mikrokristalliner Cellulose.

11. Verfahren zur Herstellung einer Tablette mit anhaltender Freisetzung nach Anspruch 8 oder 9, dadurch gekennzeichnet, daß das eindickende Verdünnungsmittel vorzugsweise ausgewählt wird aus Stärken, Stärkederivaten, Xanthangummis, mikrofeiner Cellulose und/oder Dextrinen.

12. Verfahren zur Herstellung einer Tablette mit anhaltender Freisetzung nach einem der Ansprüche 1 bis 11, dadurch gekennzeichnet, daß sie außerdem einen wäßrigen Filmüberzug auf Basis von Cellulosederivaten enthält.

13. Verfahren zur Herstellung einer Tablette mit anhaltender Freisetzung nach einem der Ansprüche 1 bis 12, dadurch gekennzeichnet, daß die Tablette außerdem Zusätze enthält, die ausgewählt werden aus Gleitmitteln (Schmiermitteln) oder färbenden Substanzen.

FIGURE   1

CINETIQUE DE DISSOLUTION

FIGURE 2

FIGURE 3

FIGURE 4